# EUROPEAN PATENT APPLICATION

(11) **EP 2 280 360 A1**
(43) Date of publication of application: **02.02.2011**
(21) Application number: 10169358.8
(22) Date of filing: 13.07.2010
(51) Int. Cl.: G06F 19/00

(54) **Device and Method for Selecting an Analyzing Instrument for use in a Medical Examination**

(30) Priority: 20.07.2009 KR 20090065976
(71) Applicant: Samsung Electronics Co., Ltd., Suwon-si, Gyeonggi-do (KR)
(72) Inventor: Kim, Hyung Chan, Gyeonggi-Do (KR)
(74) Representative: Grünecker, Kinkeldey, Stockmair & Schwanhäusser Anwaltssozietät

(57) **Abstract**

A device and a method for automatically selecting an analyzing instrument used for a medical test at medical institutions are provided.

The device includes: a receiving unit which receives information about test items; a selecting unit which searches a database and selects from the database an analyzing instrument with which a test indicated by the received information is to be performed; and a display unit which displays the analyzing instrument selected by the selecting unit.

## Description

### BACKGROUND

### 1. Field

Apparatuses and methods consistent with the exemplary embodiments relate to selecting an analyzing instrument which is used for a medical examination at medical institutes.

### 2. Description of the Related Art

Inspection and analysis of a sample collected from a patient are performed to diagnose a disease of the patient, monitor the development of the disease, determine a result of medical treatment, and determine a prognosis thereof. Several instruments and devices for analyzing a sample have recently been developed to easily perform a variety of tests on one sample at the same time. For example, in order to perform a biochemical test or an immune serum test on blood collected from a patient, an analyzing instrument, which has a chamber containing a reagent for performing the test, has been introduced. When the blood is injected into the analyzing instrument, which is then inserted to an analyzing device, the reagent contained in the chamber of the instrument is mixed with and reacts with the blood, and the result is analyzed by the device using spectroscopic methods.

In the related art, when performing such an analysis, an examiner, e.g., a medical technologist, should check the analyte that is to be examined and a test list thereof, and select an instrument for analyzing blood, which is appropriate to the test. The Examiner then injects blood collected from a patient into the instrument, and inserts the instrument into an analyzing device. During this process, however, the examiner may mistakenly select an inappropriate instrument for the test.

### SUMMARY

An exemplary embodiment provides an apparatus for automatically selecting an analyzing instrument used for medical tests at medical institutes.

Another exemplary embodiment provides a medical test device for automatically selecting an analyzing instrument used for medical tests. Another exemplary embodiment provides a medical test system which may automatically select an analyzing instrument used for medical tests.

Another exemplary embodiment provides a method for automatically selecting an analyzing instrument used for medical tests.

According to an aspect of an exemplary embodiment, there is provided an apparatus for selecting an analyzing instrument used for a medical test, the apparatus including: a receiving unit which receives information about test items; a selecting unit which searches a database and selects from the database an analyzing instrument with which a test indicated by the input information is to be performed; and a display unit which displays the analyzing instrument selected by the selecting unit.

According to an aspect of an exemplary embodiment, there is provided a medical test device which is connected through a network to a server, for selecting an analyzing instrument used for a medical test, the medical test device including: a receiving unit which receives information about test items; a selecting unit which searches a database and selects from the database an analyzing instrument with which a test indicated by the received information is to be performed; a display unit which displays the analyzing instrument selected by the selecting unit; an analyzing which performs a test on a sample contained in the analyzing instrument; and a transmitting unit which transmits to the server a result of the test performed by the analyzing unit.

According to an aspect of another exemplary embodiment, there is provided a medical test system for selecting an analyzing instrument used for a medical test, the system including a server, a terminal connected to the server through a network, and a medical test device, wherein the medical test device includes a receiving unit which receives information about test items; a selecting unit which searches a database and selects an analyzing instrument with which a test indicated by the input information is to be performed; a display unit displaying the analyzing instrument selected by the selecting unit; an analyzing unit which performs a test on a sample contained in the analyzing instrument; and a transmitting unit which transmits to the server a result of the test performed by the analyzing unit.

According to an aspect of another exemplary embodiment, there is provided a method for selecting an analyzing instrument used for a medical test, the method including inputting information about test items; searching a database and selecting from the database an analyzing instrument with which a test indicated by the input information is to be performed; and displaying the selected analyzing instrument on a display unit.

### BRIEF DESCRIPTION OF THE DRAWINGS

The above and other aspects will be more apparent from the following detailed description taken in conjunction with the accompanying drawings in which:
FIG. 1 illustrates a structure of an apparatus for selecting an analyzing instrument and of a system including the apparatus according to an exemplary embodiment;
FIG. 2 explains a structure of a database used in a method of selecting an analyzing instrument according to an exemplary embodiment;
FIG. 3 shows an example of analyzing instruments selected by the method according to an exemplary embodiment; and
FIG. 4 is a flow chart showing a method of selecting an analyzing instrument according to an exemplary embodiment.

### DETAILED DESCRIPTION OF EXEMPLARY EMBODIMENTS

Exemplary embodiments now will be described in detail with reference to the accompanying drawings as to be readily understood by a person of ordinary skill in the art. The inventive concept may, however, be embodied in many different forms and should not be construed as limited to the exemplary embodiments set forth therein. In the description, details of well-known features and techniques may be omitted to avoid unnecessarily obscuring the presented embodiments.

The terminology used herein is for the purpose of describing particular embodiments only and is not intended to limit this disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced item. The use of the terms "first", "second", and the like do not imply any particular order, but they are included to identify individual elements. Moreover, the use of the terms first, second, etc. does not denote any order or importance, but rather the terms first, second, etc. are used to distinguish one element from another. It will be further understood that the terms "comprises" and/or "comprising", or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In the drawings, like reference numerals in the drawings denote like elements.

The shape, size and regions, and the like, of the drawing may be exaggerated for clarity.

As used herein, an "analyzing instrument" may include an apparatus that performs a specific test on a biochemical sample, such as blood or urine. The instrument may be manufactured in various forms including a microfluidic chip, a chip-shaped lab-on-a-chip, a disc-shaped lab-on-a-chip, a strip-shaped device, a test tube, or other forms known to those in the art.

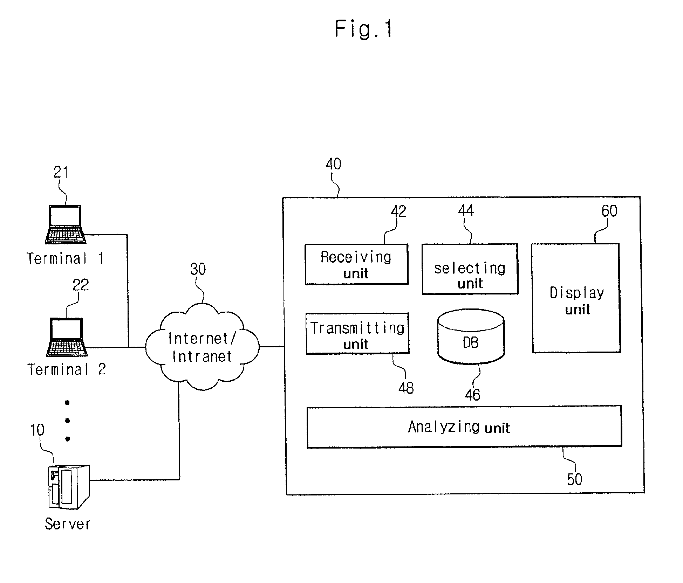

FIG. 1 illustrates a structure of an apparatus for selecting an analyzing instrument and of a medical test system including the apparatus, according to an exemplary embodiment.

The medical test system according to an exemplary embodiment includes a server 10, terminals 21 and 22 connected though a network 30 to the server 10, and apparatus 40 for selecting an analyzing instrument (hereafter "selecting apparatus").

The selecting apparatus 40 includes: a receiving unit 42 which receives information about the test items that are to be selected; an instrument selecting unit 44, which searches a database (DB) 46 and selects from the database 46 the analyzing instrument within which the test items may be performed; and a display unit 60 showing the selected instrument.

The medical test system according to an exemplary embodiment includes a server 10, a terminal 21, 22 each connected though a network 30 to the server 10, and the selecting apparatus 40.

In this exemplary embodiment, the selecting apparatus 40 is illustrated as a component of a medical test device that includes an analyzing unit 50 which performs a test on a sample contained in such an analyzing instrument, such as a blood-analyzing unit.

However, the selecting apparatus 40 according to the exemplary embodiment may be embodied as an independent apparatus separate from the medical test device. When independently provided from the medical test device, the selecting apparatus communicates with the medical test device through a network, e.g., LAN, an intranet, or the Internet.

For clarity of explanation, in an exemplary embodiment, an apparatus for selecting an analyzing instrument, or a selecting apparatus 40, may also include the medical test device within which the selecting apparatus is provided.

The selecting apparatus 40 is connected through a network 30, such as the Internet or an intranet, to the server 10. The server 10 is also connected through the network 30 to the terminals 21 and 22. The server 10 manages and stores important information including patient diagnostic records.

The receiving unit 42 of the selecting apparatus 40 receives information about test items along with a request for the test. The test item information, in the exemplary embodiment, refers to a test, such as an alanine aminotransferase (ALT) test (a type of liver function test) performed on a sample of a patient's blood, in which a particular reagent is used. However, the test item information may indicate a test, such as a liver function test, which has a broader scope than the ALT test.

The test item information may be input manually through a user interface (not shown) of the selecting apparatus 40 by an examiner, i.e., a medical technologist.

Alternatively, the test item information may be input from the terminal 21 or 22, which is connected to the server 10 through the network 30. In this case, for example, a doctor examining a patient may select test items and transmit them to the selecting apparatus 40 from a remote location, and the examiner or the technologist may receive the information and perform the tests in a more convenient way.

The selecting unit 44 of the apparatus 40 accesses the database 46, and selects an analyzing instrument available for the test corresponding to the test item information received in the receiving unit 42. Although the database 46 is included in the selecting apparatus 40 in this embodiment, but it may be located in the terminal 21 or 22, or the server 10, which is connected through the network to the apparatus 40.

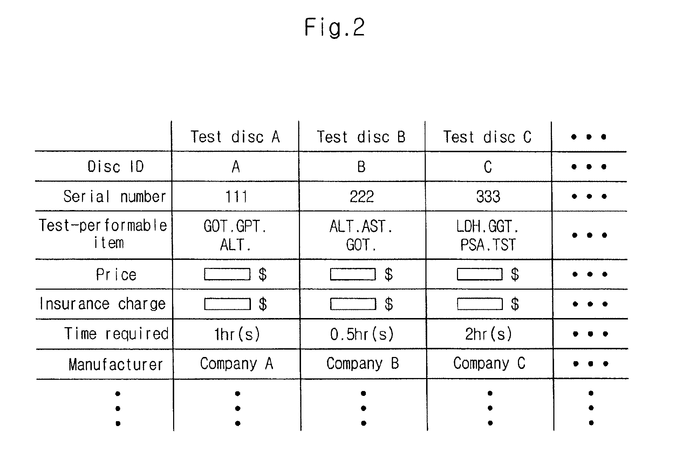

FIG. 2 illustrates a structure of the database used in a method for selecting an analyzing instrument according to an exemplary embodiment. As shown in FIG. 2, the database stores a variety of types of information about each analyzing instrument (e.g., a disc-shaped microfluidic device), including an analyzing instrument identification (ID) number, a serial number that indicates basic information about the analyzing instrument, test items that can be performed with the analyzing instrument, a price of the analyzing instrument, a medical insurance charge for the test items, an amount of time required to perform for the test, a manufacturer of the analyzing instrument, and/or other information related to the analyzing instrument.

The selecting unit 44 of the selecting apparatus 40 may search the database 46 to identify and select an analyzing instrument, with which a test corresponding to the test item information received at the receiving unit 42 may be performed. For example, when the ALT test and a glutamic-oxaloacetic transaminase (GOT) test of blood are requested, the selecting unit 44 searches the database as depicted in FIG. 2, and initially identifies test discs A and B, both of which are able to perform ALT and GOT tests.

When a plurality of analyzing instruments are identified as a result of the searching as above, the selecting unit 44 may select a single analyzing instrument by weighting different types of information associated with the analyzing instruments other than the ability to perform a specific test. In other words, the selecting unit 44 may give greater weight to a time required for a test than to a price of an instrument, a medical insurance charge, and a manufacturer of an instrument, which are all stored in the database 46, and as a result, ultimately select the test disc B, which takes a shorter time to perform the test. The weights assigned to the various types of information can be specified for example by test item information.

When the selected instrument is displayed on the display unit 60 of the selecting apparatus 40, the examiner prepares the selected instrument, injects a sample (e.g., blood) into the selected instrument, and then inserts the selected instrument containing the sample into the analyzing unit 50 of the selecting apparatus 40. The analyzing unit 50 then performs the test on the sample contained in the instrument.

Upon completion of the test, the test result is transmitted to the server 10 or the terminal 21 or 22 by a transmitting unit 48, and then is automatically stored in a database of patient records. Therefore, the selecting apparatus according to an exemplary embodiment may be adapted to operate as unit of a system such as an electronic medical record (EMR) system or a hospital information system (HIS), which automatically stores the records of patients electronically.

In an exemplary embodiment, if the examiner erroneously inserts an instrument into the analyzing unit 50 other than the selected instrument displayed on display unit 60, the selecting apparatus 40 communicating with the analyzing unit 50 may display the error on the display unit 60.

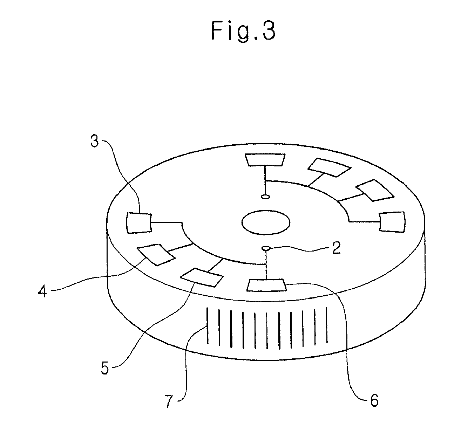

FIG. 3 is a view illustrating an example of the analyzing instruments selected in a method of selecting an analyzing instrument according to an exemplary embodiment. The analyzing instrument in this exemplary embodiment is formed as a disc rotatable by centrifugal force. A sample injected into an injecting hole 2 flows into a plurality of chambers 3, 4, 5 and 6, which contain different reagents, respectively, and a result of the reactions of the sample and the reagents is then analyzed in the analyzing unit 50 using a spectroscopic method. The analyzing unit 50 may identify a disc ID of the analyzing instrument, and the selecting unit 44 may compare the disc ID identified by the analyzing unit 50 with the disc ID of the analyzing instrument that the selecting unit 44 has selected, and may then determine whether the examiner inserted the correct instrument into the analyzing unit 50. The disc ID may be embodied as a barcode, an RFID, or other identifiers known to those skilled in the art.

Accordingly, the selecting apparatus according to the exemplary embodiment herein may prevent the examiner from committing an error during the course of a blood test.

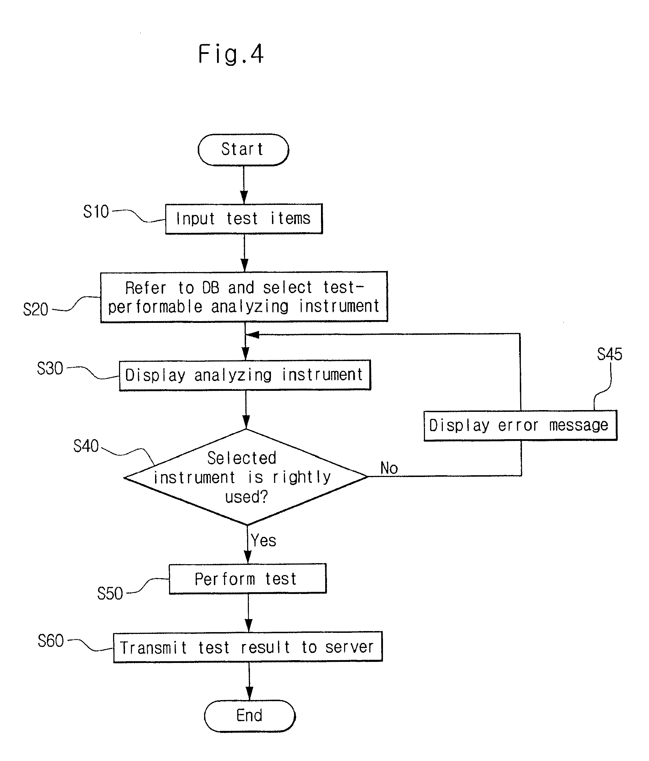

With reference to FIG. 4, a method for selecting an analyzing instrument according to an exemplary embodiment is explained below.

First, test item information is input by a doctor or an examiner (S10). The test item information may be input from an analyzing device having an analyzing unit, or from another terminal connected through a network to the device. Then, a database, such as the one shown in FIG. 2, is accessed, and searched for an analyzing instrument capable of performing the test corresponding to the test item information (S20). If a plurality of instruments are identified by the search, one of the instruments is selected by weighting different types of information regarding the plurality of instruments other than the ability to perform the test.

Then, the ID of the analyzing instrument is displayed so that the examiner may recognize the selected instrument (S30).

Next, the examiner injects a sample (e.g., blood or urine) of a patient into the selected instrument, and inserts that instrument into the analyzing unit.

It is checked whether the instrument inserted into the analyzing unit by the examiner is identical to the instrument selected at operation 20 (S40). If the examiner inserted an instrument into the analyzing unit other than the selected instrument, an error is displayed on a display unit (S45).

If it is determined that the selected instrument is inserted to the analyzing unit, the analyzing unit performs the test (S50).

Then, the result of the test is transmitted to a server or another terminal, and is automatically stored in a database of patient records (S60).

While the exemplary embodiments have been shown and described, it will be understood by those skilled in the art that various changes in form and details may be made thereto without departing from the scope of the inventive concept as defined by the appended claims.

In addition, many modifications can be made to adapt a particular situation or material to the teachings of this disclosure without departing from the essential scope thereof. Therefore, it is intended that the inventive concept not be limited to the particular exemplary embodiments disclosed, but that the inventive concept will include all embodiments falling within the scope of the appended claims.

## Claims

1. An apparatus (40) for selecting an analyzing instrument used for a medical test, the apparatus comprising:
a receiving unit (42) which receives information about test items;
a selecting unit (44) which searches a database (46) and selects from the database an analyzing instrument with which a test indicated by the received information is to be performed; and
a display unit (60) which displays the analyzing instrument selected by the selecting unit.

2. The apparatus according to claim 1, wherein the database stores database information about analyzing instruments, and the database information comprises information identifying tests the analyzing instruments are capable of performing.

3. The apparatus according to claim 2, wherein the database information further comprises at least one selected of from the group consisting of an identifier, a price, a medical insurance charge, a manufacturer of each of the analyzing instruments, and a time required for a test, in particular wherein the selecting unit assigns weights to different types of information of the database information, and selects an analyzing instrument in consideration of the weighted information.

4. The apparatus according to one of claims 1 to 3, further comprising an analyzing unit which performs a test of a sample contained in the analyzing instrument.

5. The apparatus according to one of claims 1 to 4, further comprising a transmitting unit (48) which transmits to a server (10) a result of a test performed by the selected analyzing instrument.

6. The apparatus according to one of claims 1 to 5, wherein the analyzing instrument is a microfluidic device including a chamber containing a reagent for testing a biochemical sample inserted in the microfluidic device, in particular wherein the analyzing instrument has a shape of a chip, a strip, or a disc rotatable by centrifugal force.

7. The apparatus according to one of claim 1 to 6, wherein the apparatus is communicably linked through a network (30) to a server (10).

8. The apparatus according to claim 7, wherein the information about the test items received by the receiving part is input from a terminal (21, 22) communicably linked to the server through the network.

9. The apparatus according to claim 4 or one of claims 5 to 8 in combination with claim 4, wherein the selecting unit controls the display unit to an error message if the analyzing instrument inserted into the analyzing device is different from the analyzing instrument selected by the selecting unit.

10. A method for selecting an analyzing instrument used for a medical test, the method comprising:
inputting information about test items;
searching a database and selecting from the database an analyzing instrument with which a test indicated by the input information is to be performed; and
displaying on a display unit the selected analyzing instrument.

11. The method according to claim 10, wherein the information about the test items is input through an analyzing device, a terminal connected to the analyzing device, or a server through a network.

12. The method according to claim 10 or 11, further comprising:
inserting the analyzing instrument containing a sample into an analyzing device for performing a test, and
displaying an error message on the display unit if the analyzing instrument inserted into the analyzing device is different from the selected analyzing instrument.

13. The method according to claim 10, further comprising transmitting to a server a result of the test performed by the selected analyzing instrument.

14. The method according to one of claims 10 to 13, wherein the database stores database information about analyzing instruments, and the database information comprises information identifying tests the analyzing instruments are capable of performing.

15. The method according to claim 14, wherein the database information further comprises at least one selected from the group consisting of an ID, a price, a medical insurance charge, a manufacturer of each of the analyzing instruments, and a time required for a test, in particular, wherein the selecting the analyzing instrument further comprises assigning weights to different types of information of the database information, and selecting an analyzing instrument in consideration of the weighted information.
